# EUROPEAN PATENT SPECIFICATION

(11) **EP 1 439 795 B1**
(45) Date of publication and mention of the grant of the patent: **14.06.2006**
(21) Application number: 02772519.1
(22) Date of filing: 07.10.2002
(51) Int. Cl.: A61B 19/02, B65D 77/20

(54) **ENDOSCOPE TRANSPORTATION CONTAINER**
TRANSPORTBEHÄLTER FÜR ENDOSKOPE
CAISSON DE TRANSPORT POUR ENDOSCOPE

(30) Priority: 24.10.2001 GB 0125478; 14.05.2002 US 146222
(43) Date of publication of application: 28.07.2004
(73) Proprietor: Medicart International Limited, Southend-on-Sea, Essex SS3 9QY (GB)
(72) Inventor: PARKER, George, Christopher, 40 Henley Crescent, Essex SS0 0NT (GB)
(74) Representative: Walder, Jeremy Thomas
(86) International application number: PCT/GB2002/004540
(87) International publication number: WO 2003/034936

(56) References cited:
- US-A- 3 770 119
- US-A- 4 042 109
- US-A- 5 392 917

## Description

This invention relates to a device for the in-hospital transportation of medical equipment, particularly flexible medical endoscopes, both before and after use.

A such device is known from US-A-5 392 917 and comprises a tray providing support for a medical endoscope, a tray-liner with a pouch conforming to the contours of the underlying , tray and a pouch-closing protective cover to enclose and protect the endoscope within the liner-pouch within the tray.

Flexible medical endoscopes are used for the internal examination of various parts of the human or animal body. They are produced in diameters ranging from 0.5 to 15 mm and with lengths of 300 to 3000 mm. The majority have internal channels, down which air, water or accessories may be directed so as to facilitate examinations, or to carry out surgical procedures. The modern electronic or video endoscope is a relatively expensive piece of equipment, typically costing in the region of GB £20,000.

Due to the invasive nature of many of the procedures for which flexible medical endoscopes are used, it is necessary that they be thoroughly cleaned and disinfected prior to and after each use. Ideally, the room in which the cleaning and disinfection are carried out will be in fairly close proximity to the operating theatre or procedure room. However, this ideal is seldom achieved, and this results in endoscopes being carried over fairly long distances both prior to, and after, being used on a patient.

The present invention has been developed, following experience in hospital departments carrying out flexible endoscopy over many years and in many countries, including the United Kingdom, Germany, France, Japan and the United States. The methods of carrying endoscopes between the procedure room and the cleaning room vary little between one country and another, or with the elapse of time, and would generally be considered unsatisfactory, based on such criteria as:
- protection of the endoscope both before and after use, against accidental damage or contamination;
- protection of the staff, patients, and workplace against contamination, and possible infection, deriving from a used and uncleaned endoscope; and
- protection of unused endoscopes against the potential for cross-contamination from contact with used endoscopes carrying infectious matter or with any surface that has previously been in contact with a contaminated endoscope.

Furthermore, at least in the United Kingdom and France, the recent BSE (Bovine Spongiform Encephalopathy) crisis has led to heightened concerns that the human form, Creutzfeldt-Jakob Disease (CJD), may be transmitted by contaminated endoscopes. Moreover the recent re-emergence of tuberculosis also presents a threat of airborne contamination, in areas where endoscopes are being used and transported.

The present invention seeks to address all of the above problems of moving flexible endoscopes from one area to another in a safe and hygienic way, thus without exposing anyone or anything to contamination.

The present invention has been developed particularly for use in transporting flexible medical endoscopes, and hence is described herein with particular reference to that use. Nevertheless, it will be appreciated that the present invention may also find use in the transportation of other medical equipment and/or any other situation where it is important that cleanliness be maintained or contamination be contained.

According to the present invention, there is provided a device for the in-hospital transportation of flexible medical endoscopes, both before and after use, which device comprises:
- a re-usable tray formed of a semi-rigid material which is capable of withstanding repeated disinfection, said tray having a downwardly-dished, inner endoscope compartment defined by a generally planar base, and surrounding walls upstanding therefrom, all so constructed and dimensioned as when in use to provide support for a flexible medical endoscope coiled in a substantially stress-free state therewithin, and peripheral lip-portion(s) provided at least partially around said walls and extending outwardly therefrom; and
- a single-use, disposable tray-liner formed of a flexibly deformable, sheet material substantially impermeable to bodily fluids and having margins extensive enough to be able when assembled with the underlying tray to embrace and to detachably-engage at least a major part of the lip-portion(s) thereof and be thereby removably retained thereon, said tray liner being moreover provided substantially centrally of its margins with an open-faced pouch of such a nature and/or so constructed and dimensioned that in use the tray-liner pouch is able to conform itself substantially to the contours of the dished endoscope compartment of the underlying tray; and
   either integral with or affixed to said tray liner, or even separate therefrom:

- a pouch-closing protective cover formed of material similar to that of the tray-liner which in use can be extended from one edge across the open-face of the liner pouch supported in the endoscope compartment and suitably but detachably secured to the other edge thereof so as safely to enclose and protect the endoscope when it is within the liner-pouch within the endoscope compartment.

The term "margins" as used herein refers to those portions of the liner which, when assembled with the underlying tray, are folded over the apex of the tray walls to engage with the peripheral lip-portions extending externally around the tray. The margins may conveniently be no more than upper portions of walls defining the liner pouch, or may alternatively be formed as a distinct, though still integral, component of the liner.

The dished endoscope compartment within the reusable tray may be of other regular or even irregular outline in plan view, but it is most conveniently of generally-rectangular outline.

The dimensions of the tray must be sufficient to accommodate therein substantially all sizes of flexible medical endoscope in a coiled state without undue stress being applied to the flexible portions thereof. However, the tray must also be sufficiently small to permit it to be easily carried by a person.

Therefore, it is preferred that the external dimensions of the tray are: a length of substantially 525mm, a width of substantially 425mm, and a depth of substantially 105 mm.

The corresponding internal dimensions of the endoscope compartment within the tray are preferably: a length of substantially 470mm, and a width of substantially 370mm.

So that the endoscope may be safely coiled within the endoscope compartment without any undue danger of resultant damage to its flexible parts, it is highly desirable that all the corners and intersections between the walls of the endoscope compartment and with its base shall be smoothly curved rather than sharply angular, so that they merge imperceptibly with one another and with the base of the endoscope compartment. This provides the endoscope compartment with smooth internal surfaces wherein the junctions between each wall and its next adjacent walls and also the junctions between each wall and the base are all in the form of rounded corners having a large radius of curvature.

This moreover has a further important advantage, because the fact that the junctions between the walls and with the base are all smoothly rounded eliminates any sharply angular nooks or crannies which otherwise might harbour dirt and bacteria. The cleaning and disinfection of the reusable tray are therefore greatly facilitated by thus providing it with smooth internal surfaces.

The tray is preferably formed from moulded plastics material, most preferably A.B.S. Preferably the tray is formed so as to have a sheet thickness of substantially 4mm.

The open-faced pouch in the liner needs to be such as will loosely adapt itself to the contours of the endoscope compartment in the underlying tray. The pouch itself therefore will in a sense be defined by opposed generally-vertical walls surrounding a generally- horizontal basal area roughly coextensive with the planar base of the tray. The upper ends of the walls define the open face of the pouch, where they meet with or form the margins of the tray-liner at the level of the rim of the underlying tray.

The lower ends of the side walls then come together to define the closed bottom of the pouch. It would perhaps be ideal (at least from the viewpoint of appearance) if the lower ends of the substantially vertical side walls were to be interconnected by a horizontal flat sheet of material more or less coextensive with the planar base of the underlying tray, so that the liner fits snugly within the tray compartment.

However it must be remembered that the tray-liner is a single-use, disposable thing, with little need for an aesthetic appearance. Any form of pouch that will serve its intended purpose can therefore be adopted, irrespective of aesthetics.

Bearing that in mind, it may be most appropriate to adopt a more ill-fitting construction of pouch which however is simpler and therefore more economic to manufacture. It is in fact currently preferred that the lower ends of the side walls proper should be further extended but gathered together, with suitable folding and pleating, to meet more or less centrally of the bottom of the pouch and be there integrated with one another at a junction point or more usually a junction seam.

This kind of less aesthetic but more economic construction of pouch is indeed illustrated in the accompanying drawings in relation to an endoscope compartment in the tray and correspondingly a pouch in the liner both of generally rectangular outline, and as there appears the side and end walls proper are notionally further extended downwards but in fact are folded and pleated together, to meet at a seam more or less centrally of the pouch bottom. When thus constructed and in free-hanging posture (rather than supported within the endoscope compartment of the underlying tray) of course the pouch bottom will tend to sag down, and the side and end walls will appear to taper inwards until they are gathered and integrated together into a relatively deep and rather baggy pouch.

In an alternative, and currently preferred, embodiment of the present invention, the tray-liner is made by a so-called "thermoforming" process, from a single piece of flexibly deformable sheet material, and thus has no apparent seams. Preferably, the sheet material is a plastics material, and most preferably is high density polythene (HDP).

For ease of handling, the protective cover may be pre-attached on or around the margins of the liner so as thereby to form a flap of the liner, either attached to or integral with the liner along a side of the margins corresponding to one of the walls of the pouch therein.

In this embodiment of the present invention, the pouch-closing cover of the liner may be provided with fixing means along a free side thereof, preferably the free side remote from the line of attachment/integration between liner and cover. In use, this enables said free side of the cover to be detachably secured to another side of the pouch when supported in the endoscope compartment. It is in fact greatly preferred that the cover should be provided with fixing means along at least two or even all the free sides thereof.

The fixing means may be any of any suitable kind, for instance a Zip® -fastener, a Velcro® -fastener or a press-stud closure, but these are relatively expensive for a single-use, disposable item, and so with economy in mind the fixing means is preferably an adhesive strip.

However, for reasons of economy of manufacture, amongst other things, it is generally preferred that the protective cover be provided as a separate component, to be temporarily secured at or around the margins of the liner when in use.

Where the protective cover is provided as a separate component, it should be so shaped and dimensioned as in use to embrace, and thereby be detachably secured to, the top of the endoscope compartment in a snug-fitting arrangement. This may be achieved by the provision of an elasticated skirt around the perimeter of the cover.

In a further preferred embodiment of the present invention, the protective cover is reversible and carries an indication on either side thereof of the status of the endoscope contained within the tray. In practice, this may be no more than the printing of the words "clean" and "contaminated" (or similar) on either side of the reversible cover. The reversible cover must of course be capable of being temporarily secured to an upper portion of the walls of the endoscope compartment in both its normal and reversed positions. The cover, when provided as a separate component is preferably formed of a plastics material, most preferably low density polythene (LDP).

In a still further embodiment of the present invention, there is provided a rigid lid adapted to fit snugly over the top of the protective cover when fitted on the tray, so as to provide protection against accidental piercing of the flexible cover.

Alternatively, the protective cover, or indeed the entire liner, may advantageously be formed from a self-sealing plastics material, such as a suitable grade of so-called "cling film", thus eliminating the need for the separate provision of fixing means.

The liner should be capable of co-operating with the tray in such a way that the margins thereof may be folded over externally of the endoscope compartment to engage with the peripheral lip-portion(s).

When using a generally-rectangular tray of the preferred dimensions, it is as regards the liner preferred that it should have an overall length of substantially 520mm, a width of substantially 380mm, and a depth of substantially 360mm. The liner cover then preferably has a width of substantially 510mm so as to extend across the endoscope compartment.

In a particularly preferred embodiment of the present invention, the material(s) from which the tray, the liner and/or the protective cover is/are formed will preferably be impregnated with an anti-bacterial or other biocidal or biostatic agent.

The peripheral lip-portions around the walls of the tray are intended not only for anchoring the tray liner therein but also to serve as a kind of handle for carrying the tray manually.

In a further preferred embodiment of the present invention, there is further provided a trolley having support points adapted to receive and support the peripheral lip-portion(s) of one or more trays.

In order that the present invention may more clearly be understood, one specific embodiment thereof will now be described in detail, though merely by way of example, with reference to the accompanying drawings, in which:
Figure 1 is a partly cut-away side-view of a tray having an endoscope compartment therewithin, for use in the endoscope transportation device of the present invention;
Figure 2 is a plan view from above of the tray of Figure 1;
Figure 3 is a diagrammatic side-view of a disposable liner for use in conjunction with the tray of Figures 1 and 2 in the endoscope transportation device of the present invention; and
Figure 4 is a cross-sectional view showing the tray of Figures 1 and 2 in combination with the liner of Figure 3 to form the endoscope transportation device of the present invention.

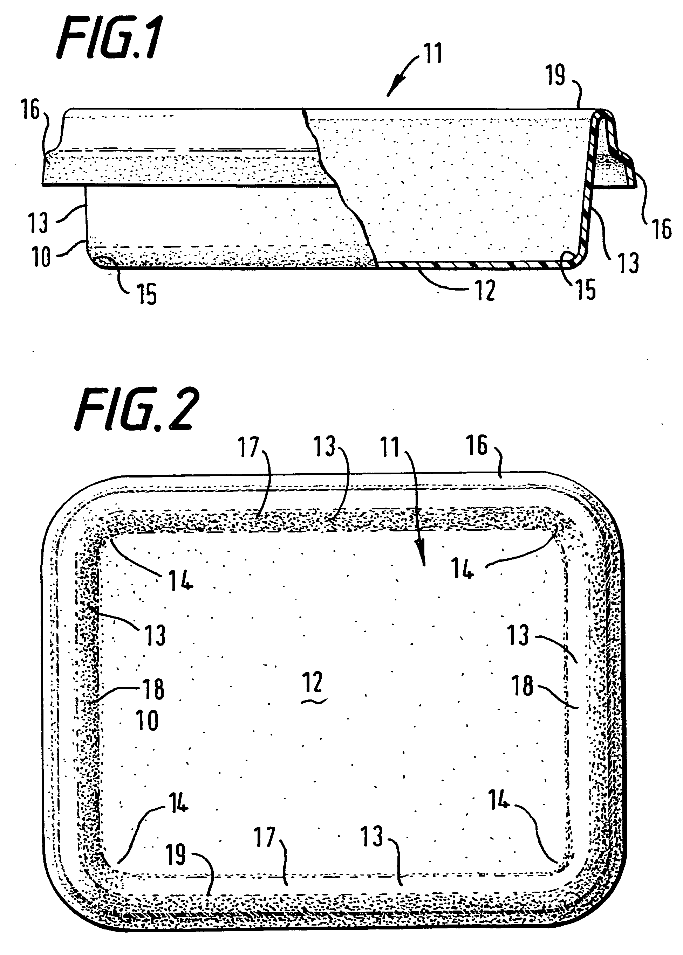

Referring first to Figures 1 and 2, there is shown a tray 10, having a downwardly-dished inner endoscope compartment, generally indicated 11 therewithin. The endoscope compartment is defined by a generally rectangular base 12 with surrounding walls 13 upstanding therefrom.

Each wall 13 is curved at each end and at the bottom thereof, such that each wall 13 merges imperceptibly with each next adjacent wall 13, and with the base 12. Thus, the junctions 14 between adjacent walls 13, and the junctions 15 between each wall and the base 12, take the form of rounded corners having a large radius. The upper edges of the end walls 13 are curled over to form a peripheral lip portion 16 externally around the walls 13. A brim 19 around the tray 10 is defined at the uppermost point of the walls 13.

As is best shown in Figure 2, the walls 13 which define the endoscope compartment 11, comprise a pair of opposed side walls 17, and a pair of opposed end walls 18. The endoscope compartment 11 is required to be large enough to accommodate substantially all sizes of flexible medical endoscope therewithin without undue stress being applied to the flexible portions thereof, yet must at the same time be small enough to permit the tray 10 to be easily carried by a person. In order to achieve this result, the side walls 17 have a length of substantially 525 mm, whilst the end walls have a length of substantially 425 mm. The depth of the endoscope compartment 11, (that is to say the height of the walls 13) is substantially 105 mm.

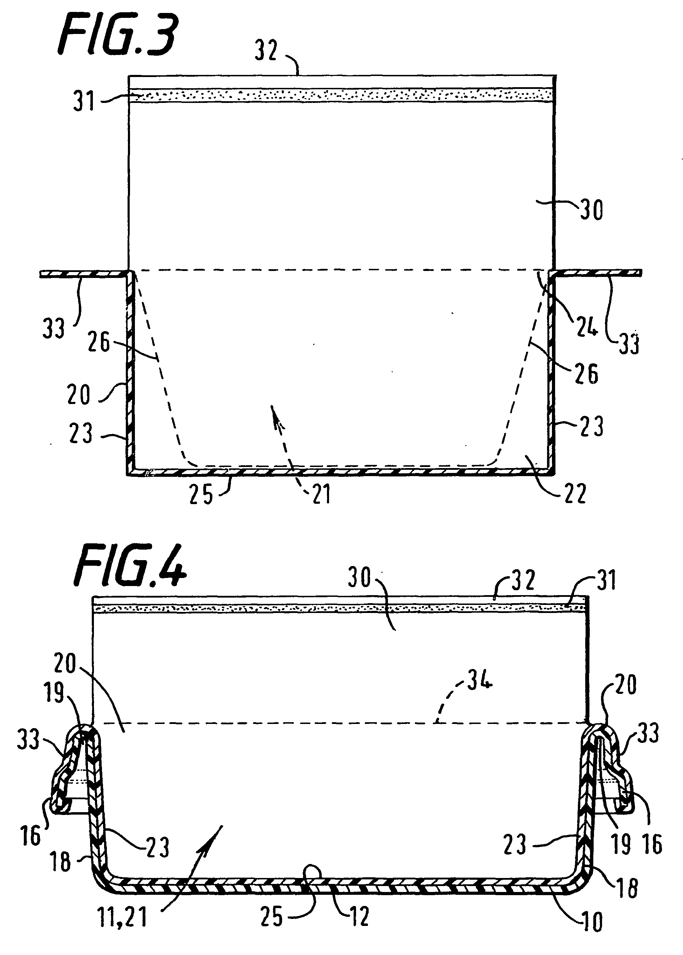

Referring now to Figure 3, there is shown a disposable liner 20 for use in conjunction with the tray 10 to form the endoscope transportation device of the present invention. The liner 20 has a pouch, generally indicated 21, defined by a pair of opposed side walls 22 and a pair of opposed end walls 23. The upper edges of the opposed side walls 22 and the opposed end walls 23 form a rim 24 defining the open end of the pouch 21, whilst the lower edges of the opposed side walls 22 meet at a seam 25 which defines the closed end of the pouch 21. The opposed end walls 23 are provided with fold lines 26, and taper inwards to meet the seam 25. However, as noted above, it is currently preferred that the liner 20 be formed so as to have no apparent seams.

The liner 20 has margins 33 adjacent the open end of the pouch 21, which although shown in Figure 3 as distinct, though still integral, components of the liner 21, may conveniently be no more than the upper portions of the walls 22, 23 defining the pouch 21.

A cover 30 is provided along a side of the rim 24 corresponding to one of the opposed side walls 22. Whilst the cover 30 in this embodiment is integral with the liner 20, for reasons of economy it may alternatively be provided as a separate component. The cover 30 is provided with an adhesive strip 31 adjacent its free end 32.

As is best shown in Figure 4, in use the liner 20 is placed into the tray 10. The margins 33 of the liner 20 are folded over the brim 19 of the tray 10, to engage with the peripheral lip 16 around the tray 10.

The liner 20 is formed of a flexibly deformable sheet material such as high density polythene, so that it may be conformed to the contours of the underlying tray 10. Thus, the pouch 21 takes on the general shape of the endoscope compartment 11, with the side walls 22 of the liner 20 corresponding to the side walls 17 of the tray 10, and the end walls 23 of the liner 20 corresponding to the end walls 18 of the tray 10. The seam 25 defining the closed end of the pouch 21 is located against the base 12 of the tray 20.

The length of the liner 20 (i.e.: the length of the side walls 22) should be substantially equal to the corresponding length of the tray 10, whilst the width of the liner (i.e.: the length of the end walls 23) should be somewhat shorter than the corresponding width of the tray 10. This enables the liner 20 to be gently stretched across the width of the tray 10, thus holding the liner 20 in a state of tension. Therefore, in the preferred embodiment, the length of the side walls 22 of the liner is substantially 520 mm, whilst the length of the end walls 23 is substantially 380 mm. The preferred depth of the liner 20 is substantially 360 mm.

The margins 33 of the liner 20, when engaged with the lip 16 of the tray 10, are located some way below the brim 19 of the tray. It is therefore necessary that the length of the cover 30 be substantially greater than the width of the tray 10. This enables the cover 30 to be folded along a fold line 34 co-incident with the brim 19 of the tray, and to extend across the width of the tray 10 to be secured on the opposed side wall 17/22 thereof, by means of the adhesive strip 31.

In use, a clean liner 20 is engaged with the tray 10 as described above. A cleaned and disinfected endoscope (not shown) may then be placed in the lined endoscope compartment 11/21. The cover 30 is then folded across the top of the lined tray 10/20 and secured at the opposite side 17/22 by means of the adhesive strip 31, so as to isolate the endoscope from any potential airborne contamination. The lined tray 10/20 with the endoscope enclosed therein may then be transported to the procedure room, either by being manually carried, using the lip 16 as a handle, or using a trolley (not shown) having connection points adapted to receive the lip 16 of one or more trays 10.

When the endoscope is to be used, the cover 30 is detached from its fixing point, and the endoscope may then be removed from the lined endoscope compartment 11/21. Once the endoscopy procedure is complete, the endoscope may be placed back into the lined endoscope compartment 11/21, and the cover 30 is re-sealed by means of the adhesive strip 31. The lined tray 10/20 is then transported back to the cleaning room, where the cover 30 is again detached from its fixing point, and the endoscope removed for cleaning and disinfecting. The used liner 20 is then removed from the tray 10, disposed of as contaminated waste, and replaced with a clean liner 20.

## Claims

1. A device for the in-hospital transportation of flexible medical endoscopes, both before and after use, which device comprises:
- a re-usable tray (10) formed of a semi-rigid material which is capable of withstanding repeated disinfection, said tray having a downwardly-dished, inner endoscope compartment (11) defined by a generally planar base (12), and surrounding walls (13) upstanding therefrom, all so constructed and dimensioned as being adapted to provide support for a flexible medical endoscope coiled in a substantially stress-free state therewithin, and peripheral lip-portion(s) (16), provided at least partially around said walls and extending outwardly therefrom; and
- a single-use, disposable tray-liner (20) formed of a flexibly deformable, sheet material substantially impermeable to bodily fluids and having margins (33) extensive enough to be able when assembled with the underlying tray to embrace and to detachably-engage at least a major part of the lip-portion(s) thereof and be thereby removably retained thereon, said tray liner being moreover provided substantially centrally of its margins with an open-faced pouch (21) of such a nature and/or so constructed and dimensioned that in use the tray-liner pouch is able to conform itself substantially to the contours of the dished endoscope compartment of the underlying tray; and
either integral with or affixed to said tray liner or even separate therefrom:
- a pouch-closing protective cover (30) formed of material similar to that of the tray-liner which is capable of being extended from one edge across the open-face of the liner pouch supported in the endoscope compartment and which is adapted to be detachably secured to another edge thereof so as safely to enclose and protect an endoscope when it is within the liner-pouch within the endoscope compartment.

2. A device as claimed in claim 1, wherein the endoscope compartment (11) within the reusable tray (10) is of generally-rectangular outline, and wherein all the corners and intersections between the walls (13) and with the base (12) of the endoscope compartment are smoothly curved so that they merge imperceptibly with one another.

3. A device as claimed in any of the preceding claims, wherein the protective cover (30) is provided as a separate component to be temporarily secured on or around the margins of the liner when in use, and so shaped and dimensioned as in use to embrace and thereby be detachably secured to an upper portion of the walls.

4. A device as claimed in claim 3, wherein said protective cover (30) is reversible, and is capable of being temporarily secured to an upper portion of said walls, in both its normal and reversed positions, and wherein said reversible cover carries on each side thereof an indication of the status of an endoscope contained within said inner compartment.

5. A device as claimed in claim 3 or claim 4, wherein said protective cover (30) is provided with an elasticated skirt, so as temporarily to secure it to an upper portion of the walls.

6. A device as claimed in claim 1 or claim 2, wherein the protective cover (30) is pre-attached on or around the margins of the liner so as thereby to form a flap of the liner, either attached to or integral with the liner along a side of the margins corresponding to one of the walls of the pouch therein.

7. A device as claimed in claim 6, wherein the flap is provided with fixing means along at least the free side remote from the line of attachment/integration between the flap and the liner, said fixing means being selected from a zip fastener, a hook and loop fastener, a press-stud closure and an adhesive strip.

8. A device as claimed in claim 6 or claim 7, wherein the flap is shaped so as in use to embrace an upper portion of the walls in a snug-fitting arrangement.

9. A device as claimed in any of the preceding claims wherein the material from which the tray and/or the liner is formed, is impregnated with an anti-bacterial agent.

10. A device as claimed in any of the preceding claims, further comprising a rigid lid adapted to fit snugly around an upper portion of the walls of said inner compartment, above said protective cover.

## Patentansprüche

1. Vorrichtung für den Transport von flexiblen medizinischen Endoskopen innerhalb eines Krankenhauses, und zwar sowohl vor und nach der Benutzung, wobei die Vorrichtung aufweist:
eine wiederverwendbare Schale (10), die aus einem halbstarren Material hergestellt ist, das in der Lage ist, einer wiederholten Desinfektion zu widerstehen, wobei diese Schale ein nach unten gerichtet tiefgezogenes, inneres Endoskop-Aufnahmefach (11), das durch eine im Wesentlichen planare Basis (12) sowie durch umgebende Wände (13) gebildet ist, die von der Basis hochstehen, die alle so konstruiert und dimensioniert sind, um ausgestaltet zu sein, um eine Aufnahme für ein flexibles medizinisches Endoskop bereitzustellen, das in einem im Wesentlichen spannungsfreien Zustand darin aufgewickelt ist, und einen Umfangs-Lippenbereich bzw. Umfangs-Lippenbereiche (16) aufweist, die zumindest teilweise um die Wände herum vorgesehen sind und sich von diesen nach außen gerichtet erstrecken; und
eine einmal verwendbare, wegwerfbare Schalen-Auskleidung (20), die aus einem elastisch verformbaren Bahnmaterial hergestellt ist, das für Körperflüssigkeiten im Wesentlichen undurchlässig ist und Randbereiche (33) hat, die ausreichend bemessen sind, um in der Lage zu sein, um nach Zusammenbau mit der darunterliegenden Schale zumindest einen wesentlichen Teil von dessen Lippenbereich (en) zu umschließen und damit lösbar einzugreifen und um **dadurch** abnehmbar daran gehalten zu sein, wobei die Schalen-Auskleidung außerdem im Wesentlichen in der Mitte bezüglich ihrer Randbereiche mit einer offenen Tasche (21) versehen ist, die eine solche Eigenschaft hat und/oder so konstruiert und dimensioniert ist, dass die Tasche der Schalen-Auskleidung bei verwendung dazu ausgestaltet ist, um sich selbst im Wesentlichen an die Konturen des tiefgezogenen Endoskop-Aufnahmefachs der darunterliegenden Schale anzupassen; und
entweder integriert mit oder an der Schalen-Auskleidung befestigt oder sogar separat davon:
eine die Tasche verschlieβende Schutzabdeckung (30), die aus einem Material hergestellt ist, das ähnlich dem der Schalen-Auskleidung ist, und die dazu ausgestaltet ist, um sich von einer Kante über die offene Seite der Tasche der Auskleidung zu erstrecken, die in dem Endoskop-Aufnahmefach gehalten ist, und die dazu ausgestaltet ist, um lösbar an der anderen Kante davon befestigt zu werden, um so ein Endoskop sicher zu umschließen und zu schützen, wenn sich dieses in der Tasche der Auskleidung in dem Endoskop-Aufnahmefach befindet.

2. Vorrichtung nach Anspruch 1, bei der das Endoskop-Aufnahmefach (11) in der wiederverwendbaren Schale (10) eine im Wesentlichen rechteckige Kontur hat, und bei der alle Ecken und Schnittstellen zwischen den Wänden (13) und der Basis (12) von dem Endoskop-Aufnahmefach leicht gekrümmt sind, so dass sie kaum merklich ineinander übergehen.

3. Vorrichtung nach einem der vorhergehenden Ansprüchen, bei der die Schutzabdeckung (30) als eine separate Komponente vorgesehen ist, um bei Benutzung temporär, an den Randbereichen der Auskleidung oder um diese herum befestigt zu werden, und so geformt und dimensioniert ist, um bei Verwendung einen oberen Bereich der Wände zu umschließen und **dadurch** abnehmbar daran befestigt zu werden.

4. Vorrichtung nach Anspruch 3, bei der die Schutzabdeckung (30) umkehrbar und dazu ausgestaltet ist, um temporär an einem oberen Bereich der Wände befestigt zu werden, und zwar in ihrer normalen und in ihrer umgekehrten Position, und bei der die umgekehrte Abdeckung an jeder Seite davon eine Angabe von dem Status eines Endoskops trägt, das in dem inneren Aufnahmefach enthalten ist.

5. Vorrichtung nach Anspruch 3 oder Anspruch 4, bei der die Schutzabdeckung (30) mit einer elastischen Schürze versehen ist, um sie temporär an einem oberen Bereich der Wände zu befestigen.

6. Vorrichtung nach Anspruch 1 oder Anspruch 2, bei der die Schutzabdeckung (30) an den Randbereichen der Auskleidung oder um diese herum vorbefestigt ist, um **dadurch** eine Klappe der Auskleidung zu bilden, die an der Auskleidung angebracht oder mit dieser integriert ist, und zwar an einer Seite der Randbereiche, die zu einer der wände von der Tasche darin gehören.

7. Vorrichtung nach Anspruch 6, bei der die Klappe mit einer Befestigungseinrichtung entlang zumi.ndest der freien Seite versehen ist, die von der Linie der Anbringung/Integration zwischen der Klappe und der Auskleidung entfernt ist, wobei die Befestigungseinrichtung ausgewählt ist aus einer Reißverschluss-Befestigungseinrichtung, einer Hacken- und Schlaufen-Befestigungseinrichtung, einer Druckknopf-Befestigungseinrichtung und einem Klebestreifen ausgewählt ist.

8. Vorrichtung nach Anspruch 6 oder Anspruch 7, bei der die Klappe so geformt ist, um bei Benutzung einen oberen Bereich der Wände in einer gut zusammenpassenden Anordnung zu umschließen.

9. Vorrichtung nach einem der vorhergehenden Ansprüche, bei der das Material, aus dem die Schale und/oder die Auskleidung hergestellt sind, mit einem antibakteriellen Mittel imprägniert ist.

10. Vorrichtung nach einem der vorhergehenden Ansprüche, außerdem mit einem starren Deckel, der dazu ausgestaltet ist, um gut zusammenpassend um einen oberen Bereich der Wände des inneren Aufnahmefachs herum zu passen, und zwar über der Schutzabdeckung.

## Revendications

1. Dispositif pour le transport d'endoscopes flexibles à l'intérieur d'un hôpital, à la fois avant et après usage, dispositif qui comprend:
- un bac réutilisable (10) formé d'un matériau semi-rigide qui est capable de supporter des désinfections répétées, ledit bac comprenant un compartiment (11) interne pour endoscope, en forme de cuvette s'étendant vers le bas, défini par une base sensiblement plane (12), et entouré de parois (13) se dressant à partir de celle-ci, toutes construites et dimensionnées de façon à être adaptées à procurer un support pour un endoscope médical flexible qui y est enroulé dans un état sensiblement sans contrainte, et un ou des élément(s) de lèvre périphérique(s) (16) disposée(s) au moins partiellement autour desdites parois et s'étendant vers l'extérieur à partir de celles-ci; et,
- une doublure (20) de bac, jetable et à usage unique, formée d'un matériau en feuille, déformable de façon flexible, étanche aux fluides corporels et ayant des rives (33) s'étendant suffisamment pour, lorsqu'elle est assemblée avec le bac qu'elle recouvre, envelopper et venir en prise détachable avec au moins une majeure partie du ou des élément(s) de lèvre périphérique(s) dudit bac, et peut y être maintenu de façon amovible, ladite doublure de bac comprenant en outre, sensiblement à l'intérieur de ses rives, une poche (21) à côté ouvert de nature telle et/ou faite et dimensionnée de telle sorte que lors de l'utilisation, la poche de la doublure de bac peut d'elle-même prendre la forme des contours du compartiment pour endoscope en forme de cuvette du bac qu'elle recouvre; et,
réalisé d'une même pièce que ladite doublure de bac ou même séparément de celle-ci:
- une couverture (30) de protection et de fermeture pour la poche réalisée dans un matériau similaire à celui de la doublure de bac, qui peut être étendue depuis un bord du côté ouvert de la poche de doublure portée dans le compartiment pour endoscope et qui est prévue pour être fixée de façon détachable à un autre bord de celle-ci de façon à enfermer et à protéger un endoscope lorsqu'il est à l'intérieur de la poche de doublure, à l'intérieur du compartiment pour endoscope.

2. Dispositif selon la revendication 1, dans lequel le compartiment pour endoscope (11) à l'intérieur du bac réutilisable (10) a une silhouette sensiblement rectangulaire, et dans lequel tous les angles et les intersections entre les parois (13) et la base (12) du compartiment pour endoscope sont doucement arrondis de façon à se fondre imperceptiblement l'un dans l'autre.

3. Dispositif selon l'une quelconque des revendications précédentes, dans lequel la couverture de protection (30) est fournie en tant que composant séparé pour être temporairement fixée sur ou autour des rives de la doublure lorsqu'elle est utilisée, et est formée et dimensionnée pour que lorsqu'elle est utilisée elle vienne envelopper et ainsi être fixée de façon détachable sur une portion supérieure des parois.

4. Dispositif selon la revendication 3, dans lequel ladite couverture de protection (30) est réversible, et peut être temporairement fixée sur une portion supérieure des parois, dans chacune de ses positions opposées, et dans lequel ladite couverture réversible porte sur chacun de ses côtés une indication du statut de l'endoscope contenu dans ledit compartiment pour endoscope.

5. Dispositif selon la revendication 3 ou la revendication 4, dans lequel ladite couverture de protection (30), comprend une jupe élastique, de façon à être temporairement fixée sur une partie supérieure des parois.

6. Dispositif selon la revendication 1 ou la revendication 2, dans lequel ladite couverture de protection (30) est préalablement attachée sur ou autour des rives de la doublure de façon à former un rabat pour la doublure, soit attaché à ou soit d'une seule pièce avec la doublure le long d'un côté d'une des rives correspondant à l'une des parois de la poche qui est à l'intérieur.

7. Dispositif selon la revendication 6, dans lequel le rabat comprend des moyens de fixation le long d'au moins trois côtés libres distants de la ligne d'attachement/raccordement entre le rabat et la doublure, lesdits moyens de fixation étant choisis parmi une fermeture éclair, une attache par complexe agrippant, une fermeture à pressions et une bande adhésive.

8. Dispositif selon la revendication 6 ou la revendication 7, dans lequel le rabat a une forme telle que lors de l'utilisation il enveloppe une portion supérieure des parois de façon parfaitement ajustée.

9. Dispositif selon l'une quelconque des revendications précédentes, dans lequel le matériau à partir duquel est formé le bac et/ou la doublure, est imprégné avec un agent anti-bactérien.

10. Dispositif selon l'une quelconque des revendications précédentes, comprenant en outre un couvercle rigide prévu pour s'ajuster parfaitement autour d'une portion supérieure des parois dudit compartiment interne, au-dessus de ladite couverture de protection.
